# EUROPEAN PATENT SPECIFICATION

(11) **EP 0 567 549 B1**
(45) Date of publication and mention of the grant of the patent: **24.06.1998**
(21) Application number: 92903968.3
(22) Date of filing: 14.01.1992
(51) Int. Cl.: A61M 29/00, A61M 29/02

(54) **DURABLE AND FLEXIBLE CATHETER OR CENTRAL LUMEN HAVING A LOW KINK RADIUS**
KATHETER ODER ZENTRALES LUMEN AUS DAUERHAFTEM UND FLEXIBLEM MATERIAL MIT NIEDRIGEM KNICKRADIUS
CATHETER DURABLE ET FLEXIBLE OU PASSAGE CENTRAL AYANT UN FAIBLE RAYON DE VRILLAGE

(30) Priority: 14.01.1991 US 640951
(43) Date of publication of application: 03.11.1993
(73) Proprietor: KONTRON INSTRUMENTS, INC., Everett, MA 02149 (US)
(72) Inventor: LOMBARDI, Edward, J., Derry, NH 03038 (US); HEGARTY, John, M., Reading, MA 01867 (US); GRIFFIN, Wayne, P., Dracut, MA 01826 (US)
(74) Representative: Riccardi, Sergio
(86) International application number: US9200410
(87) International publication number: WO9211893

(56) References cited:
- EP-A- 0 102 685
- WO-A-87/07493
- US-A- 4 282 876
- US-A- 4 306 563
- US-A- 4 762 589
- US-A- 4 960 410
- US-A- 5 041 089

## Description

### BACKGROUND OF THE INVENTION

Intra-aortic balloon (hereinafter IAB) apparatus, more particularly percutaneous IAB apparatus are now widely used for intra-aortic balloon pumping in cardiogenic shock due to acute infraction, post-operative severe low cardiac output state, or inability to wean from cardiopulmonary bypass, refractory unstable angina in the period before and after infarction, recurrent life-threatening tachyarrhythmias, and preoperative support in the present of severe left ventricular dysfunction. Additionally, intra-aortic balloon pumping has been used both experimentally and clinically to reduce infarct size. Such devices have been disclosed in U.S. 4,362,150.

One method of inserting an IAB into a patient is via a non-surgical insertion of the device through the femoral artery of the patient. This is the so-called Seldinger technique.

After insertion of the balloon catheter into the patient's femoral artery, the device must be fed through the patient's arterial system until the IAB is correctly positioned for use, usually before the subclavian artery.

This insertion process can induce trauma to the walls of the patient's arterial or venous system. A problem attendant with the insertion procedure occurs in maneuvering the device along the arterial route which may require the twisting and turning of the device.

Trauma can be minimized by providing a flexible IAB which bends in conformance with the passageways. However, there must also be a balance of flexibility and rigidity so that some degree of directional control remains and to prevent or avoid kinking.

Although flexible IAB's have been commercially available for some time, there have been numerous occurrences of central lumen failures in IAB's. Prior art central lumen include a tube made from a single homogeneous plastic while other prior art IAB's have employed a central lumen having a metal coil embedded within the wall of a plastic tube of a single homogeneous plastic material.

These and similar problems have arisen with devices which are inserted into a patient's arterial or venous system and which need to be maneuvered through the system.

EP-A-0 102 685 discloses a construction having an elongated member with two layers. However, this reference does not recognize the fact that the inner layer must be of a soft elastomeric material while the outer layer must be of a hard plastic material and that the structure is formed by coextrusion of the layers.

WO-A-87/07493 discloses a catheter comprising a tube formed by two layers of different materials but it does not recognize that the order of the layers and their formation by coextrusion lead to a durable flexible article having an unexpectedly advantageous low kink radius.

US-A-4,306,563 discloses a plastic catheter provided with a stiffening gel coating.

The deficiencies of the prior art devices are overcome and the problems solved by an intravascular catheter having the features recited in the characterizing portion of Claim 1. Further advantageous features are recited in the dependent claims.

### SUMMARY OF THE INVENTION

It is an object of the present invention to provide an improved catheter that is particularly well suited for insertion into a patient's arterial.

A further objective is to provide a durable and flexible catheter for use in an IAB or other apparatus in which a catheter is inserted into a patient's arterial system and guided therethrough.

A still further objective is to provide a catheter having a low kink radius and whose flexibility facilitates insertion of the catheter and the guiding of the catheter through tortuous arteries.

A yet further objection of the present invention is to provide a catheter which because of the low kink radius has especially good resiliency characteristics.

The foregoing and other objectives are achieved by a catheter characterized as in claim 1. In accordance with the purpose of the present invention, a catheter or central lumen is formed which comprises an elongated hollow member formed of an inner layer and an outer layer each said layer being composed of a different material and each material having its own unique chemical and physical properties. The inner layer is comprised of a soft elastomeric plastic material that imparts flexibility to the tubing. The outer layer is comprised of a hard plastic material which imparts structural support to the elongated hollow member (whether or central lumen). The combination of these two materials in a single member results in a very durable and flexible structure, exhibiting a low kink radius. Nontoxicity is achieved through the proper selection of layer materials.

Additionally, because of the low kink radius a central lumen according to the present invention has especially good resiliency characteristics. Resiliency is the restoring force which allows the central lumen to return to its original condition after, for example, a kink arises either intentionally or unintentionally during use. In the case of an IAB having a central lumen, if a kink arises, the resiliency characteristics of a central lumen according to the present invention allows the restoration to its original condition, i.e., without the kink, more quickly and more efficiently than in prior art devices.

The central lumen of the present invention is applicable to all sizes and types of double lumen IAB devices and is not limited by IAB insertion technique. It may be incorporated into IAB devices intended for insertion by the convention Seldinger technique, Sheathless technique or those techniques incorporating a tear away sheath.

In addition, the catheter or central lumen of the present invention can be used in any utility where a flexible catheter or central lumen is employed, more specifically in angioplasty, as a pulmonary artery balloon catheter, for anterograde thoracic insertion, or for catheters used with pancreatic or gall bladder problems.

The elongated hollow member of the present invention is produced by a coextrusion process. In the coextrusion process, the outer layer is formed over the inner layer using an extruder and suitable dies.

The inner layer in order to impart flexibility to the overall structure is made from a non-toxic material comprised of a soft elastomeric material, namely of polyurethanes. Pellethane 2363-65D, a polyether/polytetramethylene glycol polyurethane sold by Dow Chemicals, U.S.A. is an example of a useful polyurethane.

The outer layer in order to impart structural support to the present catheter is made from a non-toxic material comprised of a hard plastic material of nylons and preferably the hard plastic material is nylon 6.

In a preferred configuration, the catheter or central lumen of the present invention has a tubular configuration in which the inner and outer layers have constant diameters. Normally, the thickness of the outer layer is greater than the thickness of the inner layer. However, these thicknesses may be varied depending upon the application and the end result sought to be achieved. It is also possible that the diameters of the inner and outer layers may be variable either individually or together.

### BRIEF DESCRIPTION OF THE DRAWING

The features of the present invention that are believed to be novel are set forth with particularity in the appended claims. The invention, together with further objects and advantages thereof, may best be understood by reference to the following description taken in conjunction with the accompanying drawings in which:
Fig. 1 is a cut-away side elevation of the partial structure of an IAB in accordance with the invention.
Fig. 2 is a cut-away side elevation of a tubular member according to the invention.
Fig. 3 is a cut-away front elevation of a tubular member according to the invention.

### DETAIL DESCRIPTION OF THE DRAWINGS

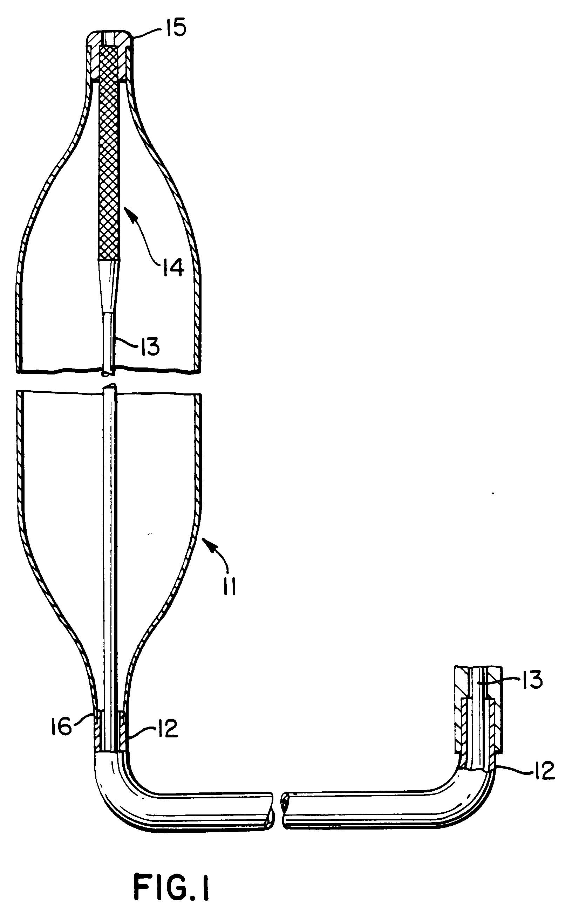

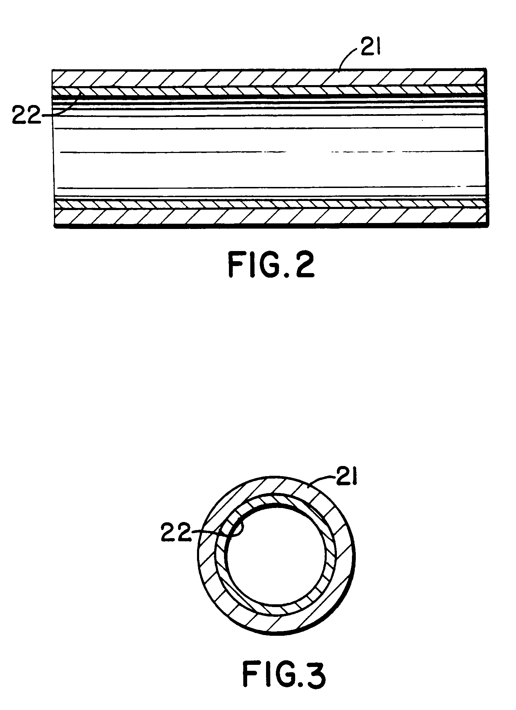

As shown in Fig. 1, a balloon catheter according to the invention includes a conventional single-chamber intra-aortic balloon 11 and a catheter 12 having disposed within in a elongated member or central lumen 13 which is more fully shown in Fig. 2 and Fig. 3 and described herein below. The balloon is illustrated by way of example as a conventional single chamber IAB.

The balloon 11 is preferably formed of an anti-thrombogenic flexible material and at is proximate end is bonded in fluid-tight manner to an end of catheter 12 and is bonded at its distal end to the flexible end 14 and elongated member or central lumen 13. Additionally, a metal ring 16 may be provided to make the balloon more easily insertible to facilitate location of the balloon. Tip 15 is sealably bonded to the distal end of the balloon.

Figures 2 and 3 show side and front elevations of elongated member or central lumen 13 of figure 1. Inner layer 22 is comprised of a soft elastomeric plastic material onto which has been coextruded the outer layer 21. Outer layer 21 is comprised of a hard plastic material.

Although only one embodiment of the present invention has been described in detail, it should be understood that the present invention may be embodied in many other specific forms without departing from the scope of the invention as claimed.

A inner layer in tubular form of polyurethane (Pellethane 2363-65D) was prepared and a layer of nylon was formed over the polyurethane tubular layer by coextrusion. This tubular material was useful as the central lumen in an IAB device and had a kink radius which is about one third that of the central lumen of typical prior art flexible balloon catheters.

The description of the preferred embodiment of the present invention is considered to be illustrative and not restrictive and the invention is not limited to the details given herein, but may be modified within the scope of the appended claims.

## Claims

1. Intravascular catheter (12) comprising a tubular body formed with an inner layer (22) and an outer layer (21), the outer layer being stiffer than the inner layer, characterized in that the inner layer (22) is being made of a material comprising a soft elastomeric plastic material and the outer layer (21) is being made of a material comprising a hard plastic material, the catheter (12) being durable and flexible and having a low kink radius, in that the inner layer (22) is a polyurethane resin and the outer layer (21) is nylon, and in that the catheter (12) is made by forming the outer layer (21) onto the inner layer (22) by coextrusion.

2. Catheter according to claim 1, characterized in that the inner layer (22) is non-toxic.

3. Catheter according to claim 1, characterized in that the outer layer (21) is non-toxic.

4. Catheter according to claim 1, characterized in that the outer layer (21) is nylon 6.

5. Catheter according to claim 1, characterized in that the catheter (12) exhibits good resiliency characteristics.

6. Balloon catheter apparatus including a hollow catheter (12) and an inflatable and deflatable balloon (11) having a proximate end and a distal end, said proximate end of the balloon (11) being sealably attached to an end of said hollow catheter (12) and an elongated member (13) which is disposed within said hollow catheter (12) and extends the length of said catheter (12) into the balloon (11), characterized in that the elongated member (13) is hollow and is comprised of the catheter (12) according to any one of claims 1 to 5.

## Patentansprüche

1. Intravaskulärer Katheter (12) mit einem rohrförmigen Körper, der durch eine innere Lage (22) und eine äußere Lage (21) gebildet wird, wobei die äußere Lage steifer als die innere Lage ist, **dadurch gekennzeichnet**, daß die innere Lage (22) aus einem Material hergestellt ist, das ein weiches elastomeres Kunststoffmaterial umfaßt, und die äußere Lage (21) aus einem Material hergestellt ist, das ein hartes Kunststoffmaterial umfaßt, wobei der Katheter (12) dauerhaft und flexibel ist und einen niedrigen Knickradius aufweist, daß die innere Lage (22) ein Polyurethanharz und die äußere Lage (21) Nylon ist, und daß der Katheter (12) durch Ausbilden der äußeren Lage (21) auf die innere Lage (22) mittels Koextrusion hergestellt ist.

2. Katheter nach Anspruch 1, **dadurch gekennzeichnet**, daß die innere Lage (22) nichttoxisch ist.

3. Katheter nach Anspruch 1, **dadurch gekennzeichnet**, daß die äußere Lage (21) nichttoxisch ist.

4. Katheter nach Anspruch 1, **dadurch gekennzeichnet**, daß die äußere Lage (21) Nylon 6 ist.

5. Katheter nach Anspruch 1, **dadurch gekennzeichnet**, daß der Katheter (12) gute Elastizitätseigenschaften aufweist.

6. Ballonkathetereinrichtung mit einem hohlen Katheter (12) und einem aufblasbaren und entleerbaren Ballon (11), der ein proximales Ende und ein distales Ende hat, wobei das proximale Ende des Ballons (11) dicht an einem Ende des hohlen Katheters (12) und einem länglichen Teil (13) befestigt ist, das innerhalb des Hohlkatheters (12) angeordnet ist und sich über die Länge des Katheters (12) hinaus in den Ballon (11) hineinerstreckt, **dadurch gekennzeichnet**, daß das längliche Teil (13) hohl ist und aus dem Katheter (12) gemäß einem der Ansprüche 1 bis 5 besteht.

## Revendications

1. Cathéter intravasculaire (12) comprenant un corps tubulaire constitué d'une couche intérieure (22) et d'une couche extérieure (21), la couche extérieure étant plus rigide que la couche intérieure, caractérisé en ce que la couche intérieure (22) est en une matière comprenant une matière plastique élastomère tendre et la couche extérieure (21) est en une matière comprenant une matière plastique dure, le cathéter (12) étant durable et flexible et ayant un faible rayon de vrillage, en ce que la couche intérieure (22) est une résine polyuréthane et la couche extérieure (21) est du nylon, et en ce que le cathéter (12) est fabriqué par formation de la couche extérieure (21) sur la couche intérieure (22) par coextrusion.

2. Cathéter suivant la revendication 1, caractérisé en ce que la couche intérieure (22) est non toxique.

3. Cathéter suivant la revendication 1, caractérisé en ce que la couche extérieure (21) est non toxique.

4. Cathéter suivant la revendication 1, caractérisé en ce que la couche extérieure (21) est du nylon 6.

5. Cathéter suivant la revendication 1, caractérisé en ce que le cathéter (12) possède de bonnes caractéristiques de résilience.

6. Dispositif de cathéter à ballonnet comprenant un cathéter creux (12) et un ballonnet gonflable et dégonflable (11) ayant une extrémité proximale et une extrémité distale, ladite extrémité proximale du ballonnet (11) étant attachée de façon étanche à une extrémité du dit cathéter creux (12), et un élément allongé (13) qui est disposé à l'intérieur dudit cathéter creux (12) et s'étend sur la longueur dudit cathéter (12) et à l'intérieur du ballonet (11), caractérisé en ce que l'élément allongé (13) est creux et est constitué du cathéter (12) suivant une quelconque des revendications 1 à 5.
